# DEMANDE DE BREVET EUROPEEN

(11) **EP 0 521 767 A1**
(43) Date de publication de la demande: **07.01.1993**
(21) Numéro de dépôt: 92401848.4
(22) Date de dépôt: 30.06.1992
(51) Int. Cl.: C12N 1/19, C12P 21/00

(54) **Procédé de production de protéines recombinantes et cellules hôtes utilisées**

(30) Priorité: 02.07.1991 FR 9108217
(71) Demandeur: RHONE-POULENC RORER S.A., 92160 Antony (FR)
(72) Inventeur: Fleer, Reinhard, F-91440 Bures Sur Yvette (FR); Fournier, Alain, F-92000 Chatenay Malabry (FR); Yeh, Patrice, F-75005 Paris (FR)
(74) Mandataire: Savina, Jacques

(57) **Abrégé**

La présente invention concerne de nouvelles souches de levure du genre Kluyveromyces lactis modifiées par génie génétique, leur préparation, et leur utilisation pour la production de protéines recombinantes.

## Description

La présente invention concerne de nouvelles souches de levure modifiées par génie génétique, leur préparation, et leur utilisation pour la production de protéines recombinantes. Plus particulièrement, l'invention concerne de nouvelles souches de levures du genre Kluyveromyces.

Il existe un grand choix d'organismes hôtes, tels que les cellules mammifères ou les microorganismes, qui peuvent potentiellement être utilisés en vue de la production de protéines recombinantes.
L'utilisation de cellules mammifères modifiées par les techniques de l'ADN recombinant présente l'avantage de conduire à des produits très proches de ceux d'origine naturelle. Cependant la culture de ces cellules est délicate, onéreuse, et ne peut être conduite que dans des volumes limités.

L'emploi de microorganismes, tels que les bactéries, permet une fabrication à une échelle plus importante mais présente l'inconvénient de conduire à des produits qui, dans certains cas, diffèrent sensiblement des produits d'origine naturelle. Ainsi les protéines normalement glycosylées chez l'homme ne sont pas, en général, glycosylées par les bactéries [P. Berman et L.A. Laskey, Trends Biochem. Sci., (1985) 10, p.51 et suivantes]. Par ailleurs, les protéines humaines exprimées à haut niveau dans des bactéries telles que E.coli acquièrent souvent une conformation non native qui s'accompagne d'une précipitation intracellulaire [R.G. Schoner et coll., Bio. Technol. (1985), 3, p.151 et suivantes; J.M. Schoemaker et coll., EMBO J. (1985), 4, p.775 et suivantes]. Enfin, pour qu'un gène puisse s'exprimer dans une bactérie, telle que E.coli, il est indispensable de positionner un codon initiateur ATG, générant une méthionine devant la séquence codante de la protéine mature. Souvent, ce résidu n'est pas excisé par la méthionyl aminopeptidase de E.coli [P.H. Seeburg et coll., 1985, 2, p.37 et suivantes ; J.M. Schoner et coll., Proc. Natl. Acad. Sci. USA (1981), 81, p.5403]. La protéine obtenue présente donc un acide aminé anormal comme premier résidu, qui peut provoquer l'inhibition stérique d'une activité biologique si le début de la protéine est impliqué dans cette activité. Le résidu peut également présenter un caractère immunogène néfaste à l'administration ultérieure de la protéine.

L'utilisation de systèmes microbiens eucaryotes tels que les levures ou les champignons représente une alternative intéressante à l'utilisation d'hôtes bactériens pour la préparation de protéines recombinantes. En effet, ces organismes possèdent toutes les caractéristiques de structure et d'organisation cellulaire des organismes eucaryotes plus complexes, tels que les cellules de mammifères. En particulier, les levures sont capables de réaliser les modifications post-transcriptionnelles et post-traductionnelles importantes pour l'activité de nombreuses protéines. En outre, les levures sont bien connues à l'echelle industrielle : elles peuvent être cultivées à haute densité cellulaire, elles ne sont pas pathogènes, ne produisent pas d'endotoxines et sont utilisées dans l'industrie alimentaire depuis très longtemps.

Les levures sont déjà utilisées comme organismes hôtes pour la production de protéines recombinantes (Cf "Yeast Genetic Engineering" Barr et al. (Eds), Butterworths, Stoneham, 1989).

En particulier, les levures du genre Saccharomyces cerevisiae ont fait l'objet de nombreuses études (voir notamment EP 60057), et les systèmes employant cette levure permettent d'exprimer à des niveaux assez élevés des gènes hétérologues. Toutefois, la capacité sécrétoire de S.cerevisiae constitue un facteur limitant dans l'exploitation de cette levure.

D'autres systèmes de production ont été développés avec les levures Pichia pastoris (EP 402 847) ou Schizosaccharomyces pombe (EP385 391), et des études sont également effectuées sur la levure Schwanniomyces (EP394 538).

Récemment, les levures du genre Kluyveromyces ont été utilisées comme organismes hôtes pour la production de protéines recombinantes. Les protéines produites par cette levure sont notamment la chymosine (EP 96 430), la thaumatine (EP 96 910), l'albumine, l'interleukine-1β, le tPA et le TIMP (EP 361 991), et des dérivés de l'albumine à fonction thérapeutique (EP 413 622).

Cependant, si des vecteurs d'expression relativement performants ont été développés pour l'utilisation de cette levure, aucune recherche n'a été effectuée dans le but d'améliorer les performances intrinsèques de la cellule utilisée. En particulier, il existe de nombreuses espèces de levures du genre Kluyveromyces, et, au sein de ces espèces, de nombreuses souches différentes. La Demanderesse a maintenant montré que ces différentes souches se comportaient de manière très hétérogène pour la production de protéines recombinantes, certaines d'entre-elles étant totalement inutilisables.

La présente invention résulte de l'identification d'une souche de levure taxonomiquement apparentée à l'espèce Kluyveromyces lactis possédant des propriétés particulièrement avantageuses pour la production de protéines recombinantes.

La présente invention décrit ainsi l'obtention de souches de levures modifiées par génie génétique, pouvant être cultivées en masse, et capables de produire efficacement, et éventuellement de sécréter dans le milieu de culture, des protéines récombinantes biologiquement actives.

Un aspect de l'invention concerne donc de nouvelles souches de levure pour la production de protéines recombinantes. Plus précisément, un objet de l'invention concerne une cellule hôte pour la production de protéines recombinantes caractérisée en ce qu'il s'agit de la levure K.lactis CBS 293.91 ou d'un dérivé ou mutant de celle-ci, contenant un fragment d'ADN hétérologue comprenant un gène de structure et des signaux permettant son expression.

Au sens de la présente invention, on entend par dérivé ou mutant, toute souche obtenue à partir de K.lactis CBS 293.91 capable d'être utilisée pour la production de protéines recombinantes. En particulier, de tels dérivés ou mutants peuvent être obtenus par modifications génétiques (altérations au niveau de l'ADN) ou biochimiques. A cet effet, différents outils de mutagénèse peuvent être utilisés, comme par exemple des outils non-spécifiques :
- agents physiques (rayons X, rayons ultra-violet ..) ou,
- agents chimiques (agents alkylants ou bialkylants, agents intercalants..),

ou des outils spécifiques tels que les systèmes d'insertions mutationnelles dirigées sur l'ADN (transposons, rétrotransposons, plasmides intégratifs, etc).

Un exemple de tels dérivés est constitué par la souche K.lactis Y616, obtenue à partir de la souche CBS 293.91 par délétion au niveau du gène URA3. D'autres mutants peuvent être obtenus par mutation au niveau de gènes codant pour des protéases, et notamment de protéases transitant par le réticulum endoplasmique telles que les protéases A, B, la carboxypeptidase Y, ou les convertases (Kex 1 en particulier).

Le fragment d'ADN hétérologue peut être introduit dans la cellule par différentes techniques. Généralement, la transformation ou l'électroporation conviennent, mais il est entendu que l'invention n'est pas limitée à une technique particulière.

Plus préférentiellement, le fragment d'ADN hétérologue comprend en outre des signaux permettant la sécrétion de la protéine recombinante. Ces signaux peuvent correspondre aux signaux naturels de sécrétion de la protéine considérée, mais ils peuvent également être d'une origine différente. En particulier des signaux de sécrétion dérivés de gènes de levure peuvent être utilisés, tels que ceux des gènes de la toxine killer (Stark et Boyd, EMBO J. 5 (1986) 1995) ou de la pheromone alpha (Kurjan et Herskowitz, Cell 30 (1982) 933; Brake et al., Yeast 4 (1988) S436).

Toujours dans un mode particulier de l'invention, le fragment d'ADN hétérologue comprend en outre un marqueur de sélection. Un tel marqueur permet en effet d'identifier facilement les cellules de l'invention. Il peut s'agir en particulier de marqueurs conférant la résistance à des antibiotiques (tel que par exemple le gène aph (Jimenez et Davies, Nature 287 (1980) 869)) ou à d'autres composés toxiques pour la cellule (ions cuivre notamment), ou de marqueurs complémentant des auxotrophies de la cellule hôte (tel que par exemple le gène URA3 (De Louvencourt et al., J.Bacteriol. 154 (1983) 737).

Généralement, les signaux permettant l'expression du gène de structure sont choisis parmi les promoteurs et les terminateurs de transcription. Il est entendu que ces signaux sont choisis en fonction du gène de structure et du résultat recherché. En particulier, il peut être préférable dans certains cas d'utiliser un promoteur régulable de manière à pouvoir découpler les phases de croissance de l'hôte et celle d'expression du gène. De même, pour des raisons de force et de compatibilité, il peut être préférable d'utiliser dans certains cas les promoteurs naturels des gènes de structure, et dans d'autres cas, des promoteurs d'origine différente.

Préférentiellement, les promoteurs utilisés sont dérivés de gènes de levure, et encore plus préférentiellement, de gènes glycolytiques de levure. D'un intérêt tout particulier sont les promoteurs dérivés de gènes glycolytiques des levures du genre Sacharomyces ou Kluyveromyces. Notamment, on peut citer les promoteurs des gènes codant pour la phosphoglycerate kinase (PGK), la glyceraldehyde-3-phosphate deshydrogenase (GPD), les énolases (ENO), ou les alcool-deshydrogenases (ADH). On peut également citer des promoteurs dérivés de gènes fortement exprimés tels que le gène de la lactase (LAC4), de la phosphatase acide (PHO5), ou des facteurs d'élongation de la traduction (TEF).

Par ailleurs, ces régions promotrices peuvent être modifiées par mutagénèse, par exemple pour ajouter des éléments supplémentaires de contrôle de la transcription, tels que notamment des régions UAS ("Upstream Activating Sequence"). A titre d'exemple, un promoteur hybride entre les promoteurs des gènes PGK et GAL1/GAL10 de S.cerevisiae donne de bons résultats.

Dans un mode préféré de réalisation de l'invention, le fragment d'ADN hétérologue fait partie d'un plasmide d'expression, qui peut être à réplication autonome ou intégratif.

S'agissant de vecteurs à réplication autonome, ils peuvent être obtenus en utilisant des séquences à réplication autonome chez K.lactis. CBS 293.91 ou ses dérivés ou mutants. En particulier, il peut s'agir de séquences chromosomiques (ARS) provenant par exemple de S.cerevisiae (Stinchcomb et al., Nature 282 (1979) 39) ou de K.lactis (Das et Hollenberg, Curr.Genet. 6 (1982) 123). Il peut également s'agir d'origines de réplication dérivées de plasmides et par exemple du plasmide pKD1 de K.drosophilarum (EP361991) ou du plasmide 2µ de S.cerevisiae (pour revue voir Futcher, Yeast 4 (1988) 27).

S'agissant de vecteurs intégratifs, ceux-ci sont généralement obtenus en utilisant des séquences homologues à certaines régions -du génome de la cellule hôte, permettant, par recombinaison homologue, l'intégration du plasmide.

Avantageusement, selon la présente invention, le gène de structure code pour une protéine d'intérêt pharmaceutique ou agroalimentaire. A titre d'exemple, on peut citer les enzymes (tels que notamment la superoxide dismutase, la catalase, les amylases, les lipases, les amidases, la chymosine etc.), les dérivés sanguins (tels que la sérum-albumine ou des variants moléculaires de celle-ci, l'alpha- ou la béta-globine, le facteur VIII, le facteur IX, le facteur von Willebrand ou certains domaines de celui-ci, la fibronectine, l'alpha-1 antitrypsine etc.), l'insuline et ses variants, les lymphokines [telles que les interleukines, les interférons, les facteurs de stimulation des colonies (G-CSF, GM-CSF, M-CSF...), le TNF, le TRF, les MIP, etc.], les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), des récepteurs viraux, ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc.)).

Préférentiellement, le gène de structure code pour la sérum-albumine humaine, un précurseur de celle-ci, ou un de ses variants moléculaires. On entend par variant moléculaire de l'albumine les variants naturels résultant du polymorphisme de l'albumine, les dérivés structuraux possédant une activité de type albumine, les formes tronquées, ou toute protéine hybride à base d'albumine.

Un autre objet de l'invention concerne un procédé de production de protéines recombinantes, selon lequel on cultive une cellule recombinée telle que définie ci-avant et on récupère la protéine produite.

Comme montré dans les exemples, ce procédé permet, de manière surprenante, d'obtenir des niveaux de production de protéines recombinantes très élevés.

Avantageusement, le procédé de l'invention permet également la sécrétion de la protéine recombinante dans le milieu de culture.

Le procédé de l'invention permet la production à des taux élevés de protéines recombinantes d'intérêt pharmaceutique ou agroalimentaire. Il est particulièrement adapté, bien que non limité, à la production de sérum-albumine humaine, ou ses variantes moléculaires.

D'autres avantages de la présente invention apparaîtront à la lecture des exemple qui suivent, qui doivent être considérés comme illustratifs et non limitatifs.

### LEGENDE DES FIGURES

Figure 1 : Construction du promoteur hybride PGK/GAL. P = promoteur; UAS = "Upstream Activating Sequence".

Figure 2 : Stratégie de construction et représentation du vecteur pYG401. P = promoteur; T = Terminateur de transcription; bla = gène conférant la résistance à l'ampicilline; aph = gène conférant la résistance à la généticine (G418).

Figure 3 : Représentation du vecteur pYG107. La légende est identique à celle de la figure 2.

Figure 4 : Expression et sécrétion d'albumine dans différentes souches de K.lactis transformées par le vecteur pYG401. Analyse en SDS-PAGE (8,5% d'acrylamide) après coloration au bleu de Coomassie, de surnageants de culture (25µl) obtenus dans les conditions décrites dans les exemples. Les lignes a et b correspondent à 0,5 et 1,0 µg d'albumine standard.

Figure 5 : Expression et sécrétion d'albumine dans différentes souches de K.lactis transformées par le vecteur pYG107. Analyse en SDS-PAGE (8,5 % d'acrylamide) après coloration au bleu de Coomassie, de surnageants de culture (25 µl) obtenus dans les conditions décrites dans les exemples. Les cellules transformées ont été cultivées en milieu M9EL10 (Cf exemple), en présence de 2 % de glucose (Glu) ou 2 % de lactose (Lac). Les lignes a et b correspondent à 0,5 et 1,0 µg d'albumine standard.

Figure 6 : Stratégie de clonage, et modification du gène URA3 de K.lactis CBS2359.

### TECHNIQUES GENERALES DE CLONAGE

Les méthodes classiques de biologie moléculaire telles que la centrifugation d'ADN plasmidique en gradient de chlorure de césium - bromure d'éthidium, la digestion par les enzymes de restriction, l'électrophorèse sur gel, l'électroélution des fragments d'ADN à partir de gels d'agarose, la transformation dans E.coli, etc, sont décrites dans la littérature (Maniatis et al., "Molecular Cloning: a Laboratory Manual", Cold Spring Harbor Laboratory, Cold Spring Harbor, N.Y., 1986 ; Ausubel et al., (eds.), "Current Protocols in Molecular Biology", John Wiley & Sons, New York 1987).

La mutagénèse dirigée in vitro par oligodésoxynucléotides est effectuée selon la méthode développée par Taylor et al. (Nucleic Acids Res. 13 (1985) 8749-8764) en utilisant le kit distribué par Amersham. Le séquençage de nucléotides est réalisé selon la technique des didéoxy décrite par Sanger et al. (Proc. Natl. Acad. Sci. USA, 74 (1977) 5463-5467). L'amplification enzymatique de fragments d'ADN spécifiques est effectuée par réaction de PCR ("Polymerase-catalyzed Chain Reaction") dans les conditions décrites par Mullis et Faloona (Meth. Enzym., (155 (1987) 335-350) et Saiki et al (Science 230 (1985) 1350-1354), en utilisant un "DNA thermal cycler" (Perkin Elmer Cetus) en suivant les recommandations du fabricant.

### EXEMPLES

### Exemple 1 : Construction de cassettes et/ou de vecteurs d'expression de protéines recombinantes.

### 1. 1. Construction de vecteurs d'expression d'albumine humaine.

### 1.1.1. Construction d'un vecteur d'expression d'albumine sous contrôle d'un promoteur hybride PGK/GAL.

Un vecteur d'expression de la sérum-albumine humaine a été préparé à partir du plasmide pYG19 (EP 361 991). Ce dernier comprend les éléments suivants :
- la séquence du plasmide pKD1, qui fait de pYG19 un plasmide à copies multiples, stable, et capable de se répliquer chez les levures du genre Kluyveromyces (EP 361 991),
- une cassette d'expression de la sérum-albumine humaine comportant le gène de structure codant pour la forme prépro sous contrôle du promoteur du gène PGK de S.cerevisiae;
- un réplicon et un marqueur de sélection gène bla conférant la résistance à l'ampicilline) bactériens ; et,
- le gène aph conférant la résistance au G418 à la levure.

Le vecteur pYG401 a été construit à partir du plasmide pYG19 par modification au niveau de la cassette d'expression de la sérum-albumine humaine. Dans pYG401, le gène de l'albumine n'est plus sous contrôle du promoteur du gène PGK de S.cerevisiae, mais sous contrôle d'un promoteur hybride entre les promoteurs des gènes pGK et GAL1/GAL10 de S.cerevisiae. Ce promoteur hybride a été obtenu en remplaçant la région UAS ("Upstream Activating Sequence") du promoteur PGK (Stanway et al., Nucl.Acid.Res. 15 (1987) 6855) par la région UAS du promoteur GAL1/GAL10 Wohnston et Davies, Mol.Cell.Biol. 4 (1984) 1440 ; West et al., Mol. Cell. Biol. 4 (1984) 2467).

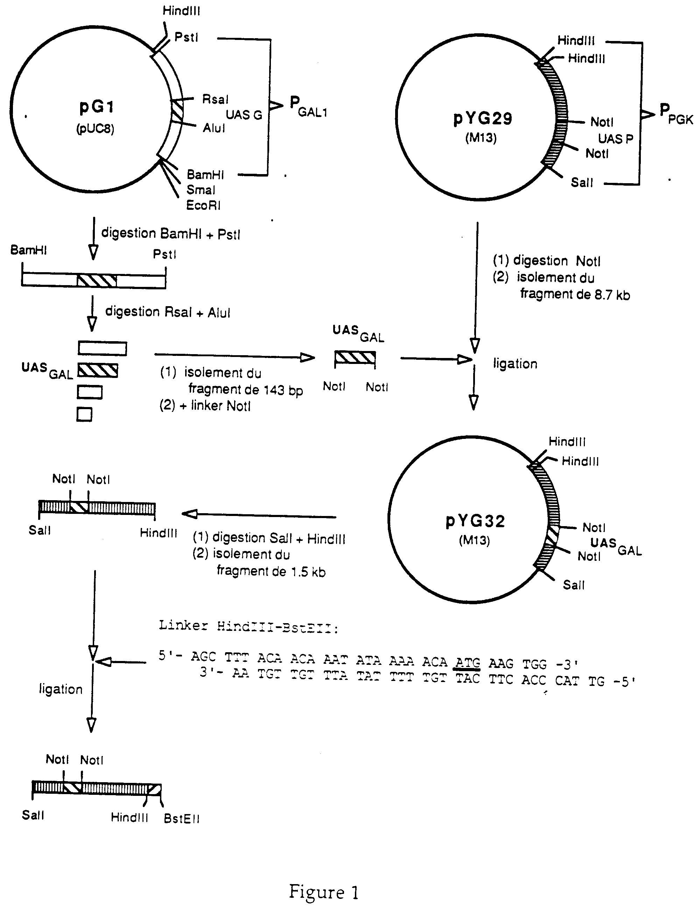

Ce promoteur hybride a été construit de la manière suivante (Cf figure 1) :
L'obtention du plasmide pYG29 a été décrite en détail dans la demande EP361 991. Ce plasmide comporte le promoteur du gène PGK de S.cerevisiae isolé à partir du plasmide pYG19 sous forme d'un fragment SalI-HindIII, et cloné dans le bactériophage M13mp18. Il a ensuite été modifié par mutagénèse dirigée pour introduire les sites de restriction suivants :
- 1 site HindIII supplémentaire en position -25 par rapport à l'ATG.
   L'introduction de ce site permet de restaurer après les différentes étapes de clonage, une région nucléotidique proche de l'ATG identique à la région native du promoteur PGK. L'environnement du codon ATG est en effet connu pour influencer de manière sensible l'efficacité de l'initiation de la traduction des gènes eucaryotes (Kozak, M., Microbiol. Rev. 47 (1983) 1-45 ; Hamilton, R., Nucl.Acid.Res 15 (1987) 3581-3593).
- 2 sites NotI de part et d'autre de la région UAS.
   L'UAS du promoteur GAL1/GAL10 a été isolée à partir du plasmide pG1 décrit par Miyajima et al. (Nucl. Acid. Res 12 (1984) 6397-6414 ; Cloning Vectors, Pouwels et al., Elsevier (1985) VI-B-ii-2). Ce plasmide est déposé à l'ATCC sous le numéro 37305.

Le plasmide pG1 comporte un fragment de 0,8 kb contenant le promoteur GAL1/GAL10 de S.cerevisiae, inséré dans le site HindII du plasmide pUC8, duquel il peut être excisé sous forme d'un fragment BamHI-PstI (figure 1).

Ce fragment a été excisé de pG1, purifié, puis digéré avec les enzymes RsaI et AluI, dont les sites de coupure respectifs sont localisés de part et d'autre de la région UAS. Un fragment de 143 pb a ainsi été isolé par electroélution, puis mis sous forme d'un fragment NotI en ajoutant un linker 5′-GCGGCCGC-3′. Ce fragment a ensuite été cloné dans le plasmide pYG29, préalablement digéré par NotI.

Le plasmide résultant est appelé pYG32 (figure 1).

Pour obtenir le vecteur d'expression portant ce promoteur hybride, le fragment SalI-HindIII portant le promoteur hybride a été isolé de pYG32'et ligaturé avec un adaptateur synthétique HindIII-BstEII composé des 2 brins complémentaires suivants: 5′-AGC TTT ACA ACA AAT ATA AAA ACA ATG AAG TGG-3′et 5′-GT TAC CCA CTT CAT TGT TTT TAT ATT TGT TGT AA-3′ (le codon d'initiation de la transcription est représenté en caractères gras). Cet adaptateur reconstitue les 22 pb situées immédiatement en amont du gène de structure PGK de S.cerevisiae, et comprend les premiers codons du gène codant pour la préproHSA, jusqu'à un site BstEII présent dans le gène natif (figure 1).

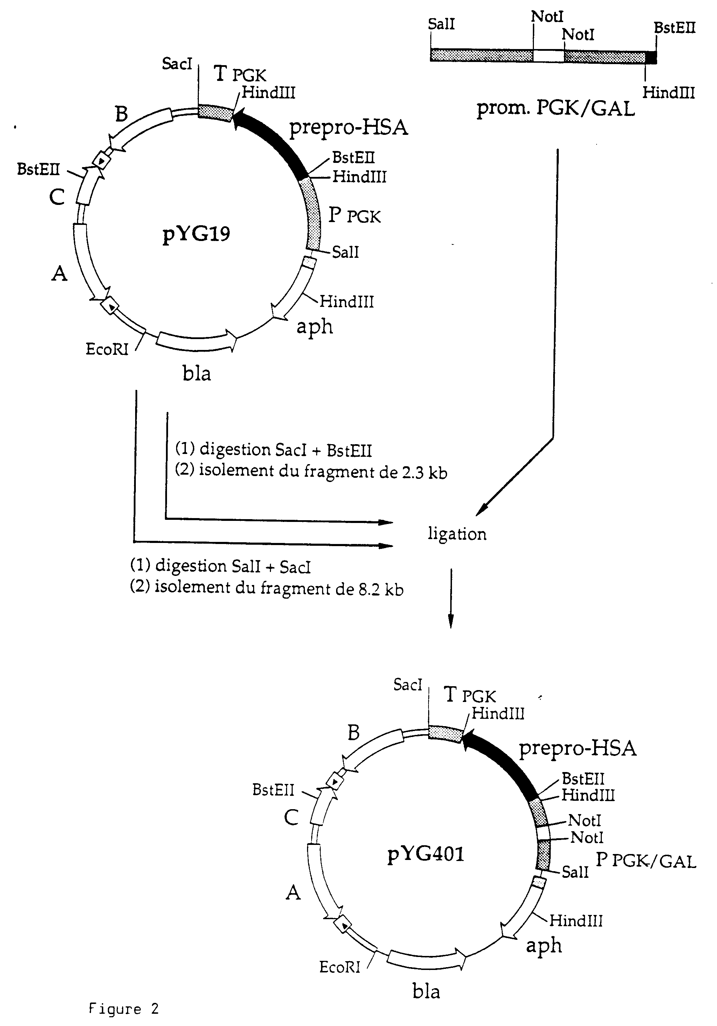

La cassette d'expression de l'albumine humaine a ensuite été reconstituée en ligaturant le fragment SalI-BstEII ainsi obtenu portant le promoteur hybride et l'extrêmité 5′ du gène de structure de l'albumine, avec le fragment BstEII-SacI isolé du plasmide pYG19, portant le reste du gène de l'albumine, et le terminateur du gène PGK de S.cerevisiae (figure 2).

La cassette ainsi obtenue a été utilisée pour remplacer la cassette d'expression SalI-SacI portée par le plasmide pYG19.

Le vecteur résultant est appelé pYG401 (figure 2).

### 1.1.2. Construction d'un vecteur d'expression d'albumine sous contrôle du promoteur LAC4 de K.lactis.

Pour tester l'efficacité du promoteur inductible LAC4 dans le système de production de la présente invention, le vecteur d'expression pYG107 a été construit. Ce vecteur est identique au vecteur pYG401 dans lequel :
(i) Le site EcoRI situé à la jonction entre la partie pKD1 et le réplicon bactérien est détruit. Cette destruction a été obtenue par coupure du plasmide pYG401 par EcoRI, remplissage des extrêmités cohésives au moyen du fragment Klenow de l'ADN polymerase de E.coli et religature.
(ii) Le site HindIII présent dans le gène aph conférant la résistance au G418 est détruit : Cette modification a été obtenue, après sous-clonage du gène aph dans le bactériophage M13mp7, par mutagénèse dirigée selon la méthode décrite par Taylor et al. (Nucleic. Acid. Res. 13 (1985) 8749), en utilisant l'oligodéoxynucléotide suivant : 5′-GAA ATG CAT AAG CTC TTG CCA TTC TCA CCG-3′. Cet oligodéoxynucléotide transforme le codon CTT codant pour la leucine 185 en CTC. Ce changement ne modifie pas la séquence protéique résultante.
   Les modifications (i) et (ii) apportent des changement mineurs, destinés uniquement à faciliter des étapes ultérieures de clonage, mais qui n'interviennent pas dans l'efficacité d'expression des vecteurs.
(iii) La cassette d'expression de l'albumine (fragment SalI-SacI) a été remplacée par la cassette SalI-SacI provenant du plasmide pYG404 (EP 361 991), dans laquelle le gène codant pour l'albumine humaine (forme prépro) est sous contrôle du promoteur LAC4 de K.lactis.

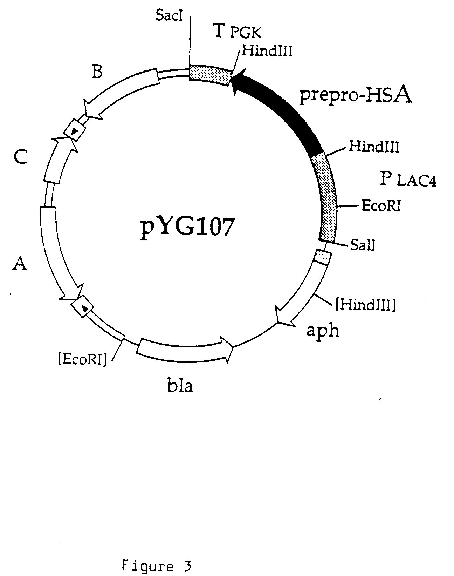

La structure du vecteur pYG107 est représentée sur la figure 3.

### Exemple 2 : Transformation de Kluyveromyces par des vecteurs d'expression de protéines recombinantes.

Différentes techniques permettant l'introduction d'un fragment d'ADN dans la levure peuvent être utilisées.

Avantageusement, les différentes souches de Kluyveromyces utilisées ont été transformées en traitant les cellules entières en présence d'acétate de lithium et de polyéthylène glycol, selon la technique décrite par Ito et al. (J. Bacteriol. 153 (1983) 163-168).

Un protocole alternatif a également été décrit en détail dans la demande EP 361 991.

### Exemple 3 : Expression et sécrétion de protéines recombinantes dans différentes levures du genre Kluyveromyces.

Cet exemple démontre que l'utilisation des nouvelles levures de l'invention modifiées génétiquement, permet d'obtenir des niveaux de production et de sécrétion de protéines recombinantes particulièrement élevés.

### 3. 1. Sérum-albumine humaine

### 3.1.1. Les souches de Kluyveromyces lactis suivantes ont été transformées par le vecteur pYG401, selon le protocole décrit dans l'exemple 2 :

- K.lactis CBS 293.91
- K.lactis CBS739
- K.lactis CBS762
- K.lactis CBS1067
- K.lactis CBS1797
- K.lactis CBS2619
- K.lactis CBS2621
- K.lactis CBS6315
- K.lactis CBS8043
- K.lactis CBS683
- K.lactis CBS579.88

La production d'albumine recombinante a été déterminée selon la méthode décrite dans la demande EP 361 991 après 120 heures de culture à 28°C sous agitation constante, en milieu YPD (Extrait de levure 10 g/l ; peptone 20 g/l ; glucose 20 g/l), en présence de généticine 2 %. Les surnageants de culture ont été obtenus après 2 centrifugations successives (5 minutes à 4000 t/min, puis 10 minutes à 12000 t/min) permettant d'éliminer toute contamination cellulaire. Un échantillon de 0,5 ml a ensuite été chauffé à 95°C pendant 15 minutes en présence d'un volume égal de tampon Tris-Hcl 0,125 M, glycérol 20 %, 2-mercaptoéthanol 10 %, sodium dodécyl sulfate (SDS) 4,6 % et 0,4 % de bleu de bromophénol (tampon Laemli 2X, Laemli, Nature 227 (1970) 680)) ; et 50 µl de la solution obtenue ont été déposés sur gel SDS-polyacrylamide 8,5 %. Après migration, le gel a été révélé au bleu de Coomassie.

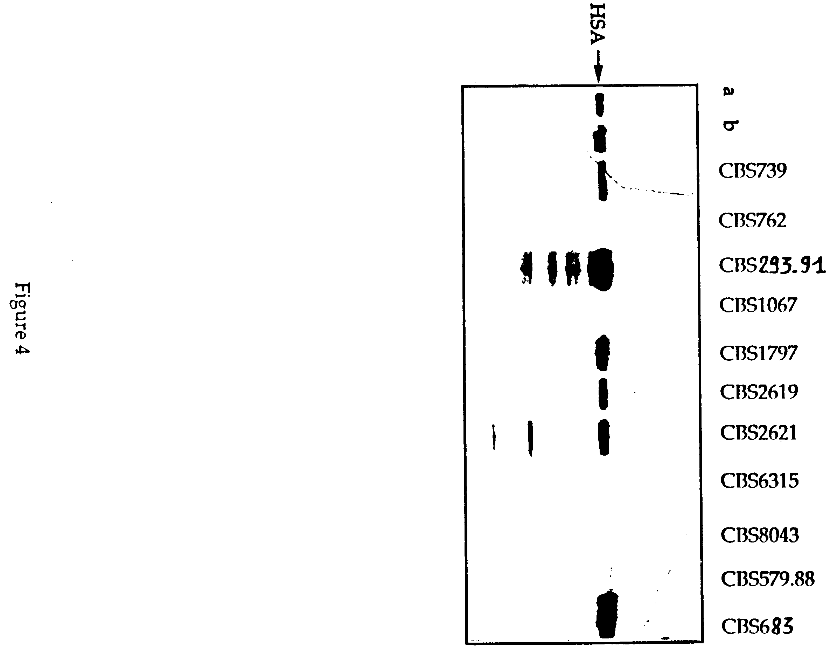

La figure 4 montre une augmentation de 200 % environ de la quantité d'albumine produite dans le système de l'invention, par rapport aux meilleurs systèmes antérieurement décrits (EP 361 991 ).

### 3.1.2. Les souches de Kluveromyces lactis suivantes ont été transformées par le vecteur pYG107, selon le protocole décrit dans l'exemple 2:

- K.lactis CBS4574
- K.lactis CBS683
- K.lactis CBS2359
- K.lactis CBS 293.91
- K.lactis CBS579.88

La production d'albumine recombinante a été déterminée selon la méthode décrite dans la demande EP 361 991 et dans l'exemple 3.1.1. après 120 heures de culture à 28°C sous agitation constante, en milieu M9EL10 en présence de glucose 20 g/l ou de lactose 20 g/l. Le milieu M9EL10 est composé du milieu M9 (Maniatis et al. précitée) supplémenté d'extrait de levure 10 g/l.

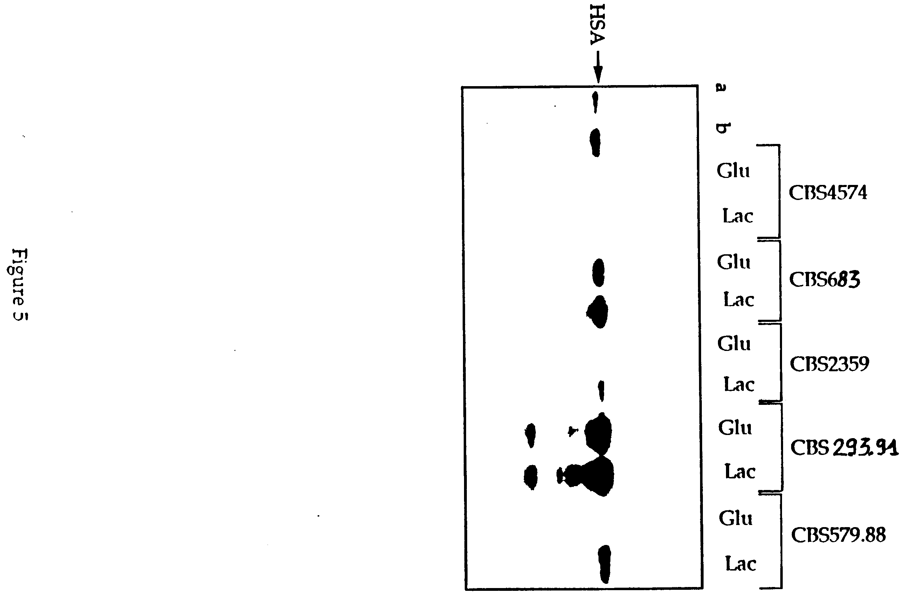

La figure 5 montre que la quantité d'albumine produite est toujours supérieure dans le système de l'invention. Elle montre également que la production est 2 à 3 fois supérieure dans un milieu contenant du lactose (inducteur du promoteur LAC4) que dans un milieu glucose. Par ailleurs, elle montre enfin que, de manière surprenante, la souche CBS 293.91 est semi-constitutive pour la production de protéines recombinantes sous contrôle du promoteur LAC4.

### 3.2. Interleukine-1β

Les souches de Kluyveromyces lactis suivantes ont été transformées par le vecteur pSPHO-IL35 (Cf EP 361 991), selon le protocole décrit dans l'exemple 2:
- K.lactis CBS683
- K.lactis CBS 293.91
- K.lactis CBS579.88

### Exemple 4: Construction d'un mutant ura3 de K.lactis CBS 293.91.

Un dérivé ura3 a été préparé à partir de K.lactis CBS 293.91. Un tel dérivé conserve les propriétés de la souche initiale, avec en plus une auxotrophie pour l'uracile qui peut être utilisée comme marqueur de sélection. Pour éviter tout événement de réversion, ce mutant a été préparé par délétion d'une partie de l'allèle URA3 chromosomique de CBS 293.91. Cette technique de mutagénèse permet également d'éviter l'emploi d'agents mutagéniques non spécifiques, pouvant altérer d'autres régions génomiques de la cellule.

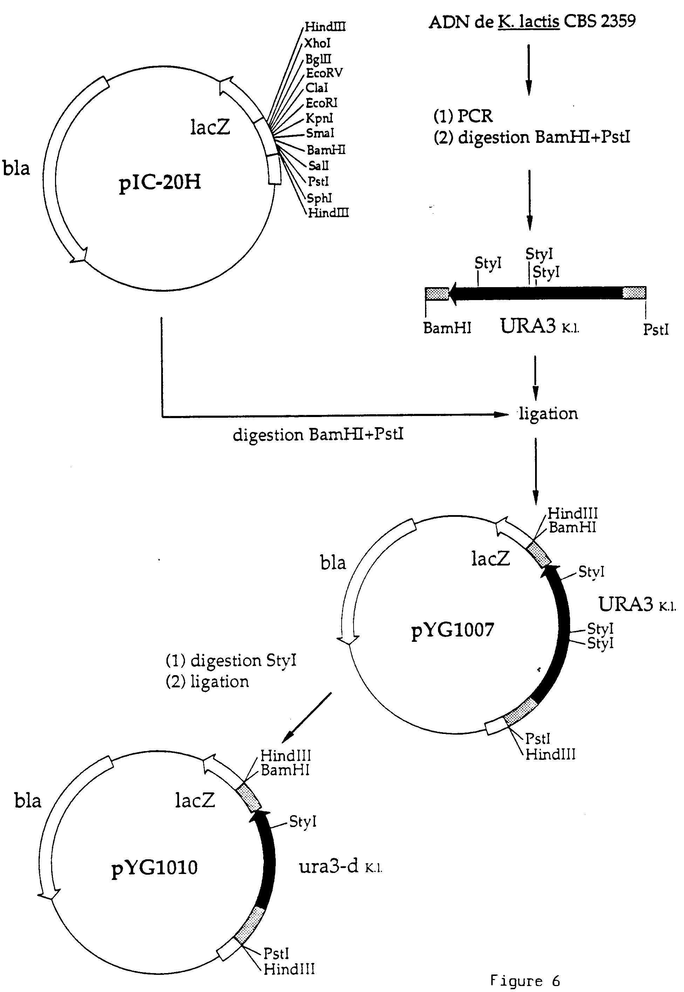

### 4.1. Clonage et modification du gène URA3 de K.lactis CBS2359 (figure 6).

Le gène URA3 de - K.lactis codant pour l'orotidine-5-phosphate decarboxylase (Shuster et al., Nucl.Acid.Res. 15 (1987) 8573) a été cloné sous forme d'un fragment BamHI-PstI de 1,2 kb en utilisant la technique de PCR (Cf techniques générales de clonage), à partir d'un extrait d'ADN génomique (Rose et al., "Methods in Yeast Genetics" Cold Spring Harbor Laboratory Press, N.Y., 1990) de - K.lactis CBS2359 au moyen des oligodéoxynucléotides suivants: 5'-GGAAGCTTGGCTGCAGGAATTGTCGTTCATGGTGACAC-3'
et

5'-
CCGAATTCCCGGATCCCATAATGAAAGAGAGAGAGAGAAGCAAAC-3'.

Le fragment obtenu a ensuite été sous-cloné dans les sites BamHI et PstI du plasmide pIC-20H (Marsh et al., Gene 32 (1984) 481) pour donner le plasmide pYG1007 (figure 6). Ce fragment a ensuite été modifié par délétion d'un fragment interne au gène URA3 comprenant 286 pb, localisées entre les sites StyI, puis religature en présence de ligase. Ce nouveau plasmide est appelé pYG1010 (figure 6).

### 4.2. Tranformation de - K.lactis CBS 293.91 par le gène URA3 délété.

La souche CBS 293.91 a été transformée par 10 µg du fragment PstI-BamHI isolé du plasmide pYG1010 par électroélution, et comportant le gène URA3, délété. Après un saut de température à 42°C ("heat shock") et 2 lavages successifs à l'eau, 600 µl de milieu YPD ont été ajoutés et les cellules ont été incubés une nuit. Les cellules ont ensuite été étalées sur milieu synthétique minimal SD ("bacto-yeast nitrogen base" sans acides aminés (Difco) 6,7 g ; glucose 20g ; Bacto-agar 20g, eau distillée 1000 ml) en présence d'uracile (100 µg/ml), d'iodine (100 µg/ml) et de 5-fluoro orotate (5FO) 15 mM.

Des clones sont apparus au bout de 4 à 5 jours. Ils ont été repiqués sur milieu YPD de manière à obtenir des colonies isolées.

A partir du 1er repiquage, 3 clones issus de la colonie initialement apparue sur milieu SD+5FO ont été réisolés sur milieu YPD (repiquage secondaire).

Les clones issus du repiquage secondaire ont ensuite été testés pour le phénotype Ura3⁻ en utilisant le test en goutte sur milieu SD et SD+uracile (Jund et Lacroute, J. of Bact. 102 (1970) 607-615 ; Bach et Lacroute, Mol. Gen. Genet. 115 (1972) 126-130). Le génotype ura3 des clones ainsi obtenus a été contrôlé par:
- réaction de PCR en utilisant les oligodéoxynucléotides décrits pour le clonage en 4.1., ce qui permet d'identifier les clones ne portant que le gène URA3 délété ;
- complémentation au moyen du plasmide pKan707 (EP 361 991) portant le gène URA3 intact de S.cerevisiae, connu pour sa capacité à complémenter l'auxotrophie ura3 chez K.lactis (De Louvencourt précitée) ; et
- Southern blot sur l'ADN génomique des clones identifés, en utilisant comme sonde le gène URA3 de K.lactis isolé dans l'exemple 4.1. marqué au ³²P selon la technique décrite par Feinberg et Vogelstein (Anal.Biochem. 132 (1983) 6).

Le mutant ura3 sélectionné est appelé K.lactis Y616.

Un échantillon de la souche K.lactis Y616 a été déposé le 11 juin 1991 auprès du Centraalbureau voor Schimmelkulturen (CBS) à Baarn aux Pays-Bas dans les conditions du Traité de Budapest, sous le numéro CBS 294.91. La souche K.lactis CBS 293'91 correspond à la souche CBS1065 redéposée le 11. juin 1991 selon les conditions du Traité de Budapest.

## Revendications

**1 -** Cellule hôte pour la production de protéines recombinantes caractérisée en ce qu'il s'agit de la levure K.lactis CBS 293.91 ou d'un dérivé ou mutant de celle-ci, contenant un fragment d'ADN hétérologue comprenant un gène de structure et des signaux permettant son expression.

**2 -** Cellule hôte selon la revendication 1 caractérisée en ce que le fragment d'ADN hétérologue comprend en outre des signaux permettant la sécrétion de la protéine recombinante.

**3 -** Cellule hôte selon la revendication 1 caractérisée en ce que le fragment d'ADN hétérologue comprend en outre un marqueur de sélection.

**4 -** Cellule hôte selon la revendication 1 caractérisée en ce que les signaux permettant l'expression du gène de structure sont choisis parmi les promoteurs et les terminateurs transcriptionnels.

**5 -** Cellule hôte selon la revendication 4 caractérisée en ce que les promoteurs transcriptionnels sont choisis parmi les promoteurs de gènes de levure, et préférentiellement de gènes glycolitiques de levure ou de gènes de levure fortement exprimés.

**6 -** Cellule hôte selon l'une quelconque des revendications 1 à 5 caractérisée en ce que le fragment d'ADN hétérologue fait partie d'un plasmide d'expression, à réplication autonome ou intégratif.

**7 -** Cellule hôte selon la revendication 6 caractérisée en ce que le fragment d'ADN hétérologue fait partie d'un plasmide d'expression à réplication autonome comprenant des séquences chromosomiques de type ARS ou une origine de réplication dérivée du plasmide pKD1 de K.drosophilarum ou du plasmide 2µ de S.cerevisiae.

**8 -** Cellule hôte selon l'une quelconque des revendications 1 à 7 caractérisée en ce que le gène de structure code pour une protéine d'intérêt pharmaceutique ou agroalimentaire.

**9 -** Cellule hôte selon la revendication 8 caractérisée en ce que la protéine est choisie parmi les enzymes (telles que notamment la superoxide dismutase, la catalase, les amylases, les lipases, les amidases, et la chymosine), les dérivés sanguins (tels que la sérum-albumine ou des variants de celle-ci, l'alpha- ou la béta-globine, le facteur VIII, le facteur IX, le facteur von Willebrand ou des domaines de celui-ci, la fibronectine, l'alpha-1 antitrypsine), l'insuline et ses variants, les lymphokines (telles que les interleukines, les interférons, les facteurs de stimulation des colonies (G-CSF, GM-CSF, M-CSF...), le TNF, le TRF,et les MIP), les facteurs de croissance (tels que l'hormone de croissance, l'érythropoiétine, le FGF, l'EGF, le PDGF, le TGF), les apolipoprotéines, des polypeptides antigéniques pour la réalisation de vaccins (hépatite, cytomégalovirus, Eppstein-Barr, herpes etc.), les récepteurs viraux, ou encore des fusions de polypeptides telles que notamment des fusions comportant une partie active fusionnée à une partie stabilisatrice.

**10 -** Cellule hôte selon la revendication 9 caractérisée en ce que la protéine est choisie parmi la sérum-albumine humaine et ses variants moléculaires.

**11 -** Procédé de production de protéines recombinantes caractérisé en ce que l'on cultive une cellule selon l'une quelconque des revendications 1 à 10 et on récupère la protéine produite.

**12 -** Procédé selon la revendication 11 caractérisé en ce que la protéine est sécrétée dans le milieu de culture.

**13 -** Procédé selon l'une des revendications 11 ou 12 pour la production d'une protéine telle que définie dans la revendication 9.

**14 -** Procédé selon la revendication 13 caractérisé en ce que la protéine est choisie parmi la sérum-albumine humaine et ses variants moléculaires.

**15 -** La levure K.lactis Y616 n° CBS 294.91.
